# FASCICULE DE BREVET EUROPEEN

(11) **EP 2 548 535 B1**
(45) Date de publication et mention de la délivrance du brevet: **04.03.2015**
(21) Numéro de dépôt: 12177013.5
(22) Date de dépôt: 19.07.2012
(51) Int. Cl.: A61F 2/46, A61B 17/16

(54) **Ancillaire d'extraction d'une prothèse**
Chirurgisches Instrument zur Entfernung einer Prothese
Ancillary device for extracting a prosthesis

(30) Priorité: 10.08.2011 FR 1157282; 19.07.2011 US 201161509506 P
(43) Date de publication de la demande: 23.01.2013
(73) Titulaire: Tornier, Inc., Bloomington, MN 55437 (US)
(72) Inventeur: Courtney, Robert Jr., Pierceton, Indiana 46562 (US); Ondrla, Jeffrey M., WARSAW, Indiana 45582 (US)
(74) Mandataire: Grand, Guillaume

(56) Documents cités:
- US-A1- 2004 186 586
- US-A1- 2006 089 656
- US-A1- 2006 195 105

## Description

La présente invention concerne un ancillaire d'extraction d'une prothèse depuis un site osseux d'implantation de cette prothèse. L'invention concerne également un ensemble chirurgical comportant un tel ancillaire et une telle prothèse.

Alors qu'une prothèse a été implantée dans un os depuis un certain temps, typiquement il y a plusieurs années, il peut s'avérer nécessaire de retirer cette prothèse en raison, entre autres, d'une usure de la prothèse, d'une dégénérescence de la matière osseuse du site d'implantation de la prothèse, d'un traumatisme, etc. Généralement, la prothèse retirée est remplacée par une prothèse dite de révision, dont le succès et les performances d'implantation dépendent du stock de matière osseuse résiduel après avoir enlevé la prothèse initiale. Par conséquent, les chirurgiens cherchent, autant que possible, à limiter les entames de la matière osseuse, nécessaires pour dégager et extraire la prothèse initiale.

Ceci étant dit, avec l'avènement des prothèses à surface poreuse ou, plus généralement, à même d'être colonisées en surface par l'os du site d'implantation, les opérations d'extraction peuvent s'avérer particulièrement délicates. Pour ce faire, le chirurgien utilise généralement des ostéotomes, dont l'application peut être avantageusement guidée pour en augmenter la précision d'action, comme proposé dans US-B-6 187 012. Puis, une fois que l'interface de solidarisation entre la prothèse et la matière osseuse a été ainsi coupée par ces ostéotomes, le chirurgien utilise un autre ancillaire pour saisir et tracter la prothèse, afin de l'extraire à proprement parler.

De son côté, US-A-2006/195 105, sur lequel est basé le préambule de la revendication 1, divulgue un ancillaire d'extraction d'une cupule de prothèse de hanche, qui est conçu pour se fixer à cette cupule soit par vissage, soit par enclenchement.

Le but de la présente invention est de proposer un ancillaire d'extraction amélioré, qui facilite et améliore les gestes chirurgicaux.

A cet effet, l'invention a pour objet un ancillaire d'extraction d'une prothèse depuis un site osseux d'implantation de cette prothèse, tel que défini à la revendication 1.

Des caractéristiques additionnelles avantageuses de cet ancillaire, prises isolément ou selon toutes les combinaisons techniquement possibles, sont spécifiées aux revendications 2 à 8.

L'invention a également pour objet un ensemble chirurgical tel que défini à la revendication 9. Une caractéristique additionnelle avantageuse de cet ensemble est spécifiée à la revendication 10.

L'invention sera mieux comprise à la lecture de la description qui va suivre, donnée uniquement à titre d'exemple et faite en se référant aux dessins sur lesquels :
- la figure 1 est une vue en perspective d'un ancillaire conforme à l'invention ;
- la figure 2 est une vue en perspective, sous un angle de vue différent et à plus grande échelle vis-à-vis de la figure 1, d'une partie de l'ancillaire de la figure 1;
- la figure 3 est une vue en élévation selon la flèche III de la figure 2 ; et
- les figures 4 à 6 sont des vues respectivement similaires aux figures 1 à 3, montrant l'ancillaire de la figure 1 associée à une prothèse à extraire à l'aide de cet ancillaire.

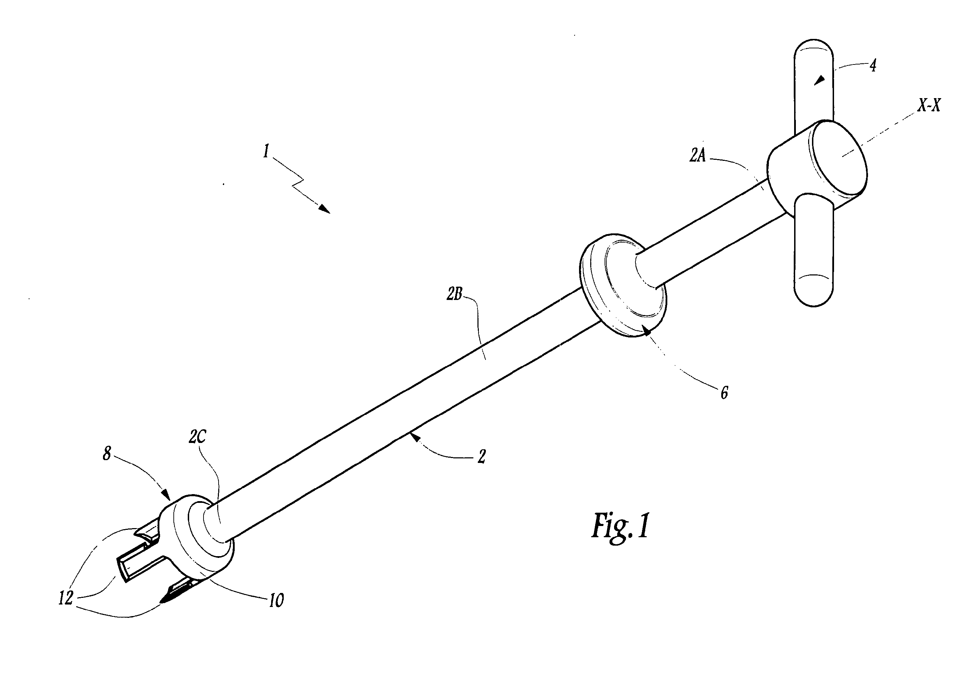

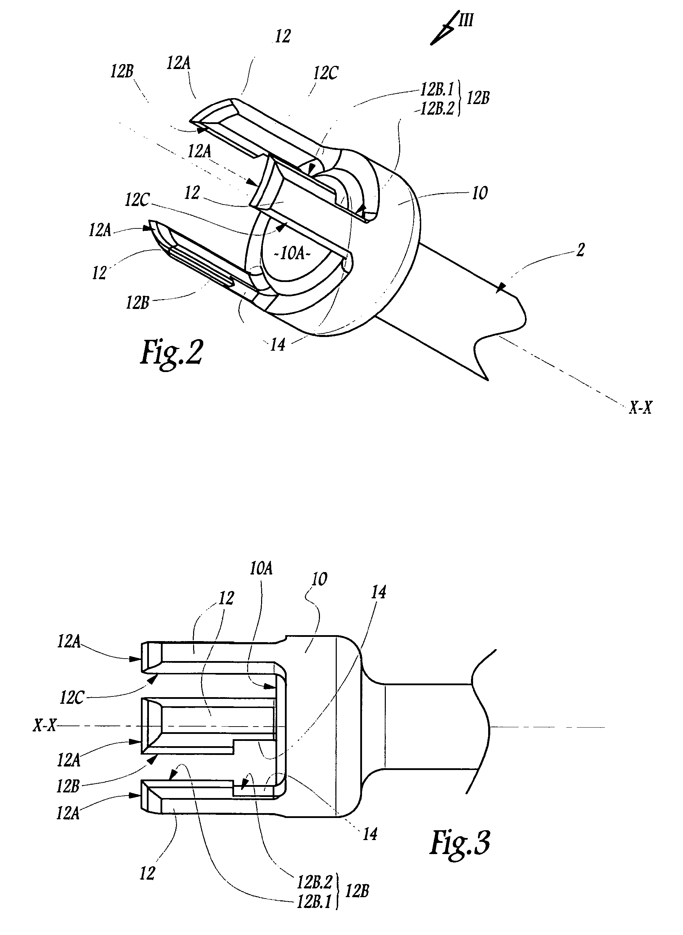

Sur les figures 1 à 3 est représenté un ancillaire 1 permettant d'extraire une prothèse depuis un site osseux d'implantation de cette prothèse. Comme bien visible sur la figure 1, cet ancillaire 1 présente une forme globale allongée, en étant centré sur un axe longitudinal X-X qui, en service, est destiné à s'étendre suivant une direction qui, côté proximal, est tournée vers le chirurgien tandis que, côté distal, cette direction est tournée vers le site osseux d'implantation de la prothèse à extraire.

L'ancillaire 1 comporte un manche 2, qui s'étend en longueur suivant et de manière centrée sur l'axe X-X et qui, dans l'exemple de réalisation considéré sur les figures, consiste en une tige cylindrique, à base circulaire.

A son extrémité proximale 2A, le manche 2 est pourvu fixement d'une poignée 4 agencée transversalement à l'axe X-X, afin de faciliter l'entraînement, notamment manuel, de l'ancillaire 1 par un utilisateur. Dans le mode de réalisation considéré sur les figures, cette poignée 4 est en forme de « T ».

Dans sa partie courante 2B, le manche 2 est fixement pourvu d'un relief 6 s'étendant radialement en saillie du reste du manche 2. Dans l'exemple de réalisation considéré ici, ce relief 6 consiste globalement en un disque centré sur l'axe X-X. L'intérêt de ce relief 6 apparaîtra plus loin.

A son extrémité 2C, le manche 2 est fixement pourvu d'une tête 8 conçue pour coopérer mécaniquement avec une prothèse à extraire. Comme bien visible sur les figures 1 à 3, cette tête 8 comporte un corps principal 10, qui est centré sur l'axe X-X et qui, dans l'exemple de réalisation considéré sur les figures, présente une forme globalement discoïdale. La tête 8 comprend également trois éléments 12, qui sont individuellement identiques les uns aux autres, tout en étant distincts les uns des autres. Plus précisément, chaque élément 12 présente une forme allongée selon la direction de l'axe X-X et s'étend longitudinalement en saillie axiale depuis la face distale 10A du corps principal 10 de la tête 8. Dans le mode de réalisation considéré ici, chaque élément 12 s'étend ainsi en saillie depuis une portion de la périphérie extérieure de la face distale 10A du corps 10. Avantageusement, chaque élément 12 correspond globalement à une portion d'une paroi tubulaire, qui serait centrée sur l'axe X-X et qui s'étendrait axialement en saillie depuis la périphérie extérieure de la face 10A du corps 10.

Les trois éléments 12 sont répartis, avantageusement de manière sensiblement régulière, autour de l'axe X-X. En particulier, dans le mode de réalisation considéré sur les figures, ces trois éléments 12 correspondent respectivement à des portions d'une même paroi tubulaire, telle que celle définie ci-dessus.

Comme représenté sur les figures 1 à 3, chaque élément 12 présente, axialement à l'opposé de son extrémité proximale reliant le reste de l'élément au corps principal 10 de la tête 8, un bord distal 12A libre, le long duquel, suivant une direction périphérique à l'axe X-X, est formée une arête coupante. De plus, suivant une direction périphérique à l'axe X-X, chaque élément 12 est délimité par deux bords longitudinaux opposés 12B et 12C libres. Le bord longitudinal 12B, qui est celui orienté dans le sens horaire autour de l'axe X-X lorsqu'on observe la tête 8 depuis l'extrémité proximale 2A du manche 2, présente, suivant sa direction longitudinale, une partie d'extrémité distale 12B.1, le long de laquelle est formée une arête coupante, et une partie d'extrémité proximale 12B.2 dans laquelle est délimitée une encoche en creux 14.

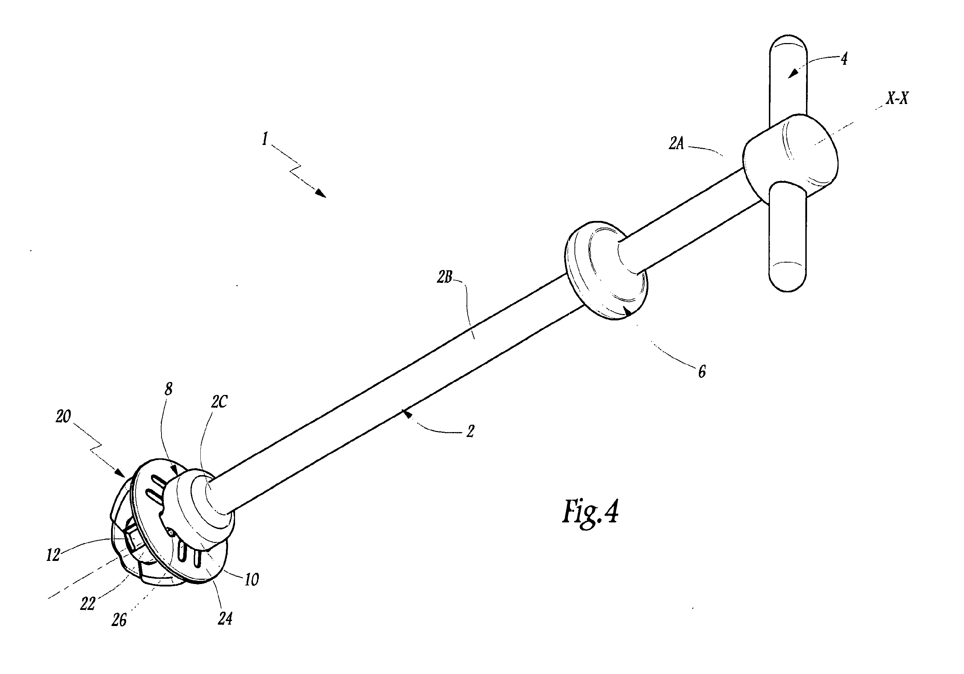

Un exemple d'utilisation de l'ancillaire 1 va maintenant être décrit en regard des figures 4 à 6.

Sur ces figures 4 à 6, la tête 8 de l'ancillaire 1 coopère avec une prothèse à extraire 20 incluant un corps 22 d'ancrage dans la matière osseuse d'un site d'implantation de la prothèse. Ce corps d'ancrage 22 est pourvu d'une collerette 24 d'appui sur le site d'implantation précité. Ainsi, on comprend que, dans la configuration d'implantation de la prothèse 20, son corps 22 est engagé en profondeur dans la matière osseuse du site d'implantation, tandis que sa collerette 24 reste émergente de la matière osseuse de ce site d'implantation, en s'appuyant sur la surface de pourtour du trou du site d'implantation à l'intérieur duquel est logé le corps 22. Par conséquent, lorsqu'un chirurgien souhaite extraire la prothèse 20 depuis le site d'implantation précité, la face proximale 24A de la collerette 24 lui est directement accessible, tandis que la face distale 24B de la collerette 24 repose en appui contre la matière osseuse du site d'implantation. Par ailleurs, eu égard au contexte de l'intervention chirurgicale, on comprend que l'interface entre la prothèse 20, plus précisément le corps 22 de cette prothèse, et la matière osseuse du site d'implantation s'avère résistante à l'extraction de la prothèse 20, dans le sens où, à la longue, un lien mécano-biologique de solidarisation s'est progressivement formé à cette interface entre la prothèse et la matière osseuse. La résistance de cette interface de solidarisation s'avère notamment particulièrement élevée dans le cas où le corps 22 présente une structure poreuse ou, plus généralement, une surface extérieure à même d'être le lieu d'une ostéo-intégration, comme c'est généralement le cas lorsque la prothèse 20 est une prothèse implantée sans ciment.

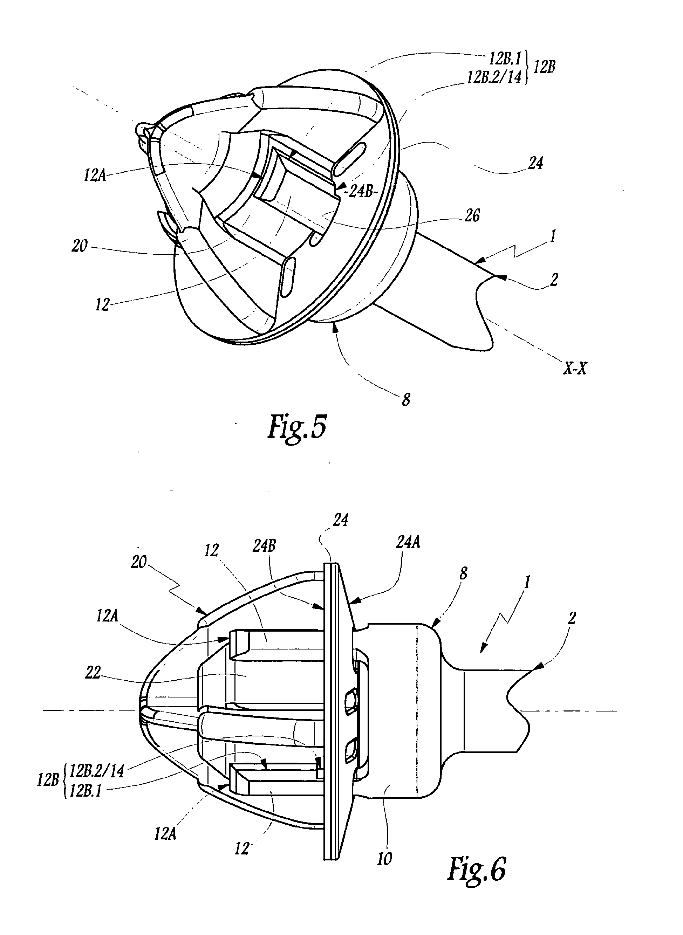

Pour extraire la prothèse 20, le chirurgien se saisit, notamment manuellement, du manche 2 de l'ancillaire 1 et rapproche la tête 8 de la prothèse 20, en alignant sensiblement l'axe X-X avec un axe géométrique central de la prothèse 20, en particulier l'axe géométrique central autour duquel la colorette 24 s'étend de manière périphérique. Le bord distal 12A de chaque élément 12 de la tête 8 va pouvoir alors être utilisé pour couper au moins en partie l'interface de solidarisation entre la prothèse 20 et la matière osseuse du site d'implantation. Pour ce faire, la collerette 24 est pourvue de trois fentes traversantes 26 qui, chacune, relient l'une à l'autre les faces proximale 24A et distale 24B de la collerette 24. Chaque fente 26 présente une section transversale autorisant, voire avantageusement guidant l'introduction dans cette fente de l'un des éléments 12, suivant un mouvement de translation orienté selon l'axe X-X et dirigé dans le sens distal. Dans l'exemple de réalisation considéré sur les figures, cela revient à dire que chaque fente 26 présente une section correspondant à une portion d'anneau plat, dont la largeur, considérée radialement à l'axe X-X, correspond sensiblement à l'épaisseur radiale de chaque élément 12, et dont la longueur, suivant une direction périphérique à l'axe X-X, est sensiblement égale à l'étendue périphérique de l'élément 12, comme bien visible sur les figures 4 et 5.

Bien entendu, c'est le bord distal 12A des éléments 12 qui est d'abord introduit dans une des fentes 26 et qui en débouche, du côté distal, en coupant la portion de l'interface de solidarisation entre la prothèse 20 et la matière osseuse qu'il rencontre sur sa trajectoire de translation. Le chirurgien maintient l'entraînement translatif de l'ancillaire 1 selon l'axe X-X, jusqu'à ce que la face distale 10A du corps 10 vienne en appui contre ou à proximité immédiate de la face proximale de la prothèse 20, notamment de la périphérie intérieure de la face proximale 24A de la collerette 24.

Le chirurgien entraîne alors le manche 2 en rotation sur lui-même autour de l'axe X-X, avantageusement en s'aidant de la poignée 4 pour décupler le couple d'entraînement. La tête 8 est alors entraînée suivant un mouvement rotatif identique, conduisant le bord longitudinal 12B de chacun de ses éléments 12 à suivre une trajectoire circulaire, centrée sur l'axe X-X et, dans l'exemple de réalisation considéré ici, en sens horaire. La partie d'extrémité distale 12B.1 de chacun des bords 12B coupe alors la partie de l'interface de solidarisation entre le corps 22 de la prothèse 20 et la matière osseuse du site d'implantation, partie située sur la trajectoire circulaire suivie par le bord 12B. Dans le même temps, chaque encoche 14 des bords longitudinaux 12 met en prise mécanique la collerette 24, dans le sens où, eu égard au mouvement rotatif de la tête 8 sur elle-même autour de l'axe X-X par rapport à la prothèse 20, une des extrémités périphériques de chaque fente 26 se retrouve introduite dans l'encoche 14, plus précisément en s'engageant, suivant une direction périphérique à l'axe X-X, axialement entre les bords axiaux opposés de l'encoche 14. Ce mouvement d'entraînement en rotation est poursuivi par le chirurgien, de manière à engager la collerette 24 jusque dans le fond des encoches 14. L'ancillaire 1 et la prothèse 20 sont alors dans la configuration d'utilisation représentée sur les figures 4 à 6.

Au vu des explications qui précèdent, on comprend que la liaison mécanique que le chirurgien vient d'établir entre la tête 8 de l'ancillaire 1 et la collerette 24 de la prothèse 20 est une liaison à baïonnette, centrée sur l'axe X-X.

Dans un troisième temps opératoire, le chirurgien exerce un effort de traction suivant l'axe X-X, dirigé dans le sens proximal. Avantageusement, le chirurgien s'aide du relief 6, notamment en engageant mécaniquement ce relief 6 avec un outil ad hoc, non représenté sur les figures, lui permettant de décupler son effort pour appliquer au manche 2 des contraintes de traction axiale dirigées dans le sens proximal. Comme l'interface de solidarisation entre la prothèse 20 et la matière osseuse du site d'implantation de cette prothèse a été coupée en plusieurs zones, sous l'action successive des bords distaux 12A et des parties d'extrémité distale 12B.1 des bords longitudinaux 12B des éléments 12, les zones non coupées restantes de cette interface de solidarisation se rompent de manière contrôlée quant à leur emplacement, ainsi que facilement, sans que le chirurgien ait à exercer un trop grand effort de traction.

Ainsi, l'ancillaire 1 permet d'extraire facilement et rapidement la prothèse 20, étant remarqué que, grâce à sa fixation à baïonnette, intégrant des coupes partielles de l'interface de solidarisation entre la prothèse et la matière osseuse du site d'implantation, le chirurgien n'a pas à utiliser deux instruments distincts pour, tour à tour, couper l'interface précitée puis accrocher mécaniquement la prothèse à tracter.

Bien entendu, divers aménagements optionnels peuvent être apportés à l'ancillaire 1 décrit jusqu'ici.

## Revendications

1. Ancillaire (1) d'extraction d'une prothèse (20) depuis un site osseux d'implantation de cette prothèse, cet ancillaire définissant un axe proximo-distal (X-X) et comportant une tête d'extrémité distale (8) adaptée pour, à la fois, couper au moins partiellement l'interface de solidarisation entre la prothèse et la matière osseuse du site d'implantation, et se fixer à la prothèse,
**caractérisé en ce que** la tête d'extrémité distale (8) est adaptée pour se fixer à la prothèse (20) par une liaison à baïonnette centrée sur l'axe proximo-distal (X-X).

2. Ancillaire suivant la revendication 1, **caractérisé en ce que** la tête d'extrémité distale (8) comprend au moins un élément allongé (12), qui s'étend en longueur suivant une direction sensiblement parallèle à l'axe proximo-distal (X-X) et qui présente un bord distal libre (12A) coupant, ainsi qu'un bord longitudinal libre (12B) à la fois coupant et pourvu d'une encoche de baïonnette (14) pour mettre en prise mécaniquement une partie dédiée (24) de la prothèse (20).

3. Ancillaire suivant la revendication 2, **caractérisé en ce que** l'encoche de baïonnette (14) est située dans une partie d'extrémité proximale (12B.2) du bord longitudinal associé (12B) de l'élément (12).

4. Ancillaire suivant l'une des revendications 2 ou 3, **caractérisé en ce que** le ou chaque élément (12) est porté par la tête d'extrémité distale (8) de manière à, d'une part, lorsque la tête d'extrémité distale est translatée le long de l'axe proximo-distal (X-X) par rapport à la prothèse à extraire (20), couper une partie de ladite interface de solidarisation par le bord distal (12A) de l'élément et, d'autre part, lorsque la tête d'extrémité distale (8) est ensuite entraînée en rotation sur elle-même autour de l'axe proximo-distal (X-X) par rapport à la prothèse à extraire (20), à la fois couper une autre partie de ladite interface de solidarisation par le bord longitudinal (12B) de l'élément et engager la partie dédiée (24) de la prothèse dans l'encoche de baïonnette (14) de ce bord longitudinal.

5. Ancillaire suivant l'une quelconque des revendications 2 à 4, **caractérisé en ce que** le ou chaque élément (12) correspond à une portion d'une paroi tubulaire centrée sur l'axe proximo-distal (X-X).

6. Ancillaire suivant l'une quelconque des revendications 2 à 5, **caractérisé par** plusieurs éléments (12) distincts, qui s'étendent chacun en saillie, suivant l'axe proximo-distal (X-X), d'une même face distale (10A) du corps principal (10) de la tête d'extrémité distale (8), en étant répartis suivant une périphérie extérieure de cette face distale.

7. Ancillaire suivant l'une quelconque des revendications précédentes, **caractérisé en ce que** l'ancillaire (1) comporte en outre un manche (2), qui est centré sur l'axe proximo-distal (X-X), dont l'extrémité distale (2C) est fixement pourvue de la tête d'extrémité distale (8), et dont l'extrémité proximale (2A) est fixement pourvue d'une poignée d'entraînement (4).

8. Ancillaire suivant la revendication 7, **caractérisé en ce que** le manche (2) est pourvu, dans sa partie courante (2B), d'un relief (6) d'application au manche d'un effort de traction, orienté selon la direction de l'axe proximo-distal (X-X) et dirigé vers l'extrémité proximale (2A) du manche.

9. Ensemble chirurgical, comprenant :
- un ancillaire (1) conforme à l'une quelconque des revendications précédentes, et
- une prothèse d'implantation osseuse (20), qui est pourvue d'une collerette (24) d'appui sur le site osseux d'implantation de la prothèse, cette collerette étant adaptée pour être fixée à la tête d'extrémité distale (8) de l'ancillaire (1) par la liaison à baïonnette.

10. Ensemble suivant la revendication 9, **caractérisé en ce que** l'ancillaire (1) est conforme à la revendication 2 ou à l'une des revendications dépendantes de la revendication 2, et **en ce que** la collerette (24) de la prothèse (20) est pourvue d'au moins une fente traversante (26), qui relie la face (24B) de la collerette, en appui sur le site d'implantation, à sa face opposée (24A), et qui est adaptée pour être traversée, avantageusement de manière guidée, par l'élément (12) appartenant à la tête d'extrémité distale (8) de l'ancillaire (1).

## Patentansprüche

1. Hilfsvorrichtung (1) zur Extraktion einer Prothese (20) von einem Knochen-Implantations-Standort dieser Prothese, wobei diese Hilfsvorrichtung eine proximal-distale Achse (X-X) definiert und einen Distalendkopf (8) aufweist, der angepasst ist, um zugleich wenigstens teilweise die Verbindungsschnittstelle zwischen der Prothese und dem Knochenmaterial des Implantationsstandorts zu durchtrennen und sich an der Prothese zu fixieren,
**dadurch gekennzeichnet, dass** der Distalendkopf (8) angepasst ist, um sich an der Prothese (20) zu fixieren mittels einer zur proximal-distalen Achse (X-X) zentrierten Bajonett-Verbindung.

2. Hilfsvorrichtung gemäß Anspruch 1, **dadurch gekennzeichnet, dass** der Distalendkopf (8) wenigstens ein langgestrecktes Element (12) aufweist, das sich in Längsrichtung entlang einer zur proximal-distalen Achse (X-X) im Wesentlichen parallelen Richtung erstreckt und das eine freie distale schneidende Kante (12A) sowie einen freien Längsrand (12B) aufweist, der zugleich schneidend und mit einer Bajonettaussparung (14) versehen ist, um einen spezifischen Abschnitt (24) der Prothese (20) mechanisch zu ergreifen.

3. Hilfsvorrichtung gemäß Anspruch 2, **dadurch gekennzeichnet, dass** die Bajonettaussparung (14) in einem proximalen Endabschnitt (12B.2) des zugehörigen Längsrands (12B) des Elements (12) angeordnet ist.

4. Hilfsvorrichtung gemäß einem der Ansprüche 2 oder 3, **dadurch gekennzeichnet, dass** das oder jedes Element (12) in einer Weise von dem Distalendkopf (8) getragen ist, um einerseits, wenn der Distalendkopf entlang der proximal-distalen Achse (X-X) bezüglich der zu extrahierenden Prothese (20) translationsbewegt wird, einen Teil der besagten Verbindungsschnittstelle mittels der distalen Kante (12A) des Elements zu durchtrennen und um andererseits, wenn der Distalendkopf (8) anschließend seinerseits um die proximal-distale Achse (X-X) rotationsangetrieben wird bezüglich der zu extrahierenden Prothese (20), zugleich einen anderen Teil der besagten Verbindungsschnittstelle mittels des Längsrands (12B) des Elements zu durchtrennen und den spezifischen Abschnitt (24) der Prothese in der Bajonett-Aussparung (14) dieses Längsrands zu ergreifen.

5. Hilfsvorrichtung gemäß irgendeinem der Ansprüche 2 bis 4, **dadurch gekennzeichnet, dass** das oder jedes Element (12) einem Abschnitt einer zu der proximal-distalen Achse (X-X) zentrierten Rohrwand entspricht.

6. Hilfsvorrichtung gemäß irgendeinem der Ansprüche 2 bis 5, **gekennzeichnet durch** mehrere individuelle Elemente (12), die sich jeweils entlang der proximal-distalen Achse (X-X) von einer selben Distalstirnseite (10A) des Hauptkörpers (10) des Distalendkopfs (8) vorstehend erstrecken, wobei sie entlang einer Außenperipherie dieser Distalstirnseite verteilt sind.

7. Hilfsvorrichtung gemäß irgendeinem der vorhergehenden Ansprüche, **dadurch gekennzeichnet, dass** die Hilfsvorrichtung (1) ferner einen Schaft (2) aufweist, der zur proximal-distalen Achse (X-X) zentriert ist, dessen distales Ende (2C) fest mit dem Distalendkopf (8) versehen ist und dessen proximales Ende (2A) fest mit einem Antriebsgriff (4) versehen ist.

8. Hilfsvorrichtung gemäß Anspruch 7, **dadurch gekennzeichnet, dass** der Schaft (2) in seinem Verlaufsabschnitt z) versehen ist mit einer Erhebung (6) zur Aufbringung einer in Richtung der proximal-distalen Achse (X-X) orientierten und zum proximalen Ende (2A) des Kanals hin gerichteten Zugbelastung auf den Schaft.

9. Chirurgischer Kit, aufweisend:
- eine Hilfsvorrichtung (1) gemäß irgendeinem der vorgehenden Ansprüche und
- eine Knochen-Implantations-Prothese (20), die mit einem Kragen (24) zur Auflage am Knochen-Implantations-Standort der Prothese versehen ist, wobei dieser Kragen angepasst ist, um an dem Distalendkopf (8) der Hilfsvorrichtung (1) mittels der Bajonett-Verbindung fixiert zu sein.

10. Kit gemäß Anspruch 9, **gekennzeichnet dadurch, dass** die Hilfsvorrichtung (1) gemäß Anspruch 2 ist oder gemäß irgendeinem der Ansprüche ist, die von Anspruch 2 abhängen, und dadurch, dass der Kragen (24) der Prothese (20) mit wenigstens einem Durchgangsschlitz (26) versehen ist, der die in Auflage am Implantationsstandort befindliche Seite (24B) des Kragens mit deren gegenüberliegenden Seite (24A) verbindet und der angepasst ist, um, vorteilhaft in geführter Weise, von dem Element (12) durchdrungen zu sein, welches dem Distalendkopf (8) der Hilfsvorrichtung (1) angehört.

## Claims

1. Ancillary device (1) for extraction of a prosthesis (20) from a site of implantation of that prosthesis in bone, the ancillary device defining a proximo-distal axis (X-X) and having a distal end head (8) arranged both to at least partially cut the interface of union between the prosthesis and the bony material of the implantation site and to attach to the prosthesis,
**characterised in that** the distal end head (8) is arranged to attach to the prosthesis (20) by means of a bayonet connection centred on the proximo-distal axis (X-X).

2. Ancillary device according to claim 1, **characterised in that** the distal end head (8) comprises at least one elongate element (12) which extends length-wise in a direction substantially parallel to the proximo-distal axis (X-X) and which has a free distal edge (12A) which cuts and also a free longitudinal edge (12B) which both cuts and is provided with a bayonet notch (14) for mechanically engaging a dedicated part (24) of the prosthesis (20).

3. Ancillary device according to claim 2, **characterised in that** the bayonet notch (14) is located in a proximal end part (12B.2) of the associated longitudinal edge (12B) of the element (12).

4. Ancillary device according to one of claims 2 or 3, **characterised in that** the distal end head (8) bears the or each element (12) in such a way that, on the one hand, when the distal end head is moved in translation along the proximo-distal axis (X-X) relative to the prosthesis (20) to be extracted, it cuts a part of said union interface by means of the distal edge (12A) of the element and, on the other hand, when the distal end head (8) is then driven in rotation about itself, around the proximo-distal axis (X-X), relative to the prosthesis (20) to be extracted, it both cuts another part of said union interface by means of the longitudinal edge (12B) of the element and engages the dedicated part (24) of the prosthesis in the bayonet notch (14) of that longitudinal edge.

5. Ancillary device according to any one of claims 2 to 4, **characterised in that** the or each element (12) corresponds to a portion of a tubular wall centred on the proximo-distal axis (X-X).

6. Ancillary device according to any one of claims 2 to 5, **characterised by** a plurality of separate elements (12), each of which projects out, along the proximo-distal axis (X-X), from the same distal face (10A) of the main body (10) of the distal end head (8), being distributed on an outer periphery of that distal face.

7. Ancillary device according to any one of the preceding claims, **characterised in that** the ancillary device (1) additionally includes a shaft (2) centred on the proximo-distal axis (X-X), the distal end (2C) of which is fixedly provided with the distal end head (8) and the proximal end (2A) of which is fixedly provided with a driving handle (4).

8. Ancillary device according to claim 7, **characterised in that** the shaft (2) is provided, in its extending part (2B), with a projection (6) for application of a traction force to the shaft, which is oriented according to the direction of the proximo-distal axis (X-X) and directed towards the proximal end (2A) of the shaft.

9. Surgical system comprising:
- an ancillary device (1) in accordance with any one of the preceding claims, and
- a bone implant prosthesis (20) provided with a collar (24) in abutment against the implantation site of the prosthesis in bone, that collar being arranged to become attached to the distal end head (8) of the ancillary device (1) by means of the bayonet connection.

10. System according to claim 9, **characterised in that** the ancillary device (1) is in accordance with claim 2 or with one of the claims dependent on claim 2; and **in that** the collar (24) of the prosthesis (20) is provided with at least one through-slot (26) which connects that face (24B) of the collar which is in abutment against the implantation site to its opposite face (24A) and which is arranged for the element (12) belonging to the distal end head (8) of the ancillary device (1) to pass through it, advantageously in guided manner.
